(19) Europäisches Patentamt
European Patent Office
Office européen des brevets

(11) Publication number: 0 243 153
A2

(12) **EUROPEAN PATENT APPLICATION**

(21) Application number: 87303509.1

(22) Date of filing: 22.04.87

(51) Int. Cl.³: C 12 P 21/02
C 12 N 15/00

(30) Priority: 22.04.86 US 856643
14.11.86 US 931458

(43) Date of publication of application:
28.10.87 Bulletin 87/44

(84) Designated Contracting States:
AT BE CH DE ES FR GB GR IT LI LU NL SE

(71) Applicant: IMMUNEX CORPORATION
51 University Street Suite 600 600 Immunex Building
Seattle Washington 98101(US)

(72) Inventor: Cerretti, Douglas Pat
1607 North 197th Place
Seattle Washington 98133(US)

(72) Inventor: Cosman, David John
116 11th Avenue East, No. 301
Seattle Washington 98102(US)

(72) Inventor: Gillis, Stephen
3455 West Mercer Way
Mercer Island Washington 98040(US)

(72) Inventor: MOchizuki, Diane Yukiko
4805 Stanford Avenue Northeast
Seattle Washington 98105(US)

(72) Inventor: March, Carl Jack
8133 8th Southwest
Seattle Washington 98106(US)

(72) Inventor: Price, Virginia Lee
2617 Boyer Avenue East
Seattle Washington 98102(US)

(72) Inventor: Tushinski, Robert J.
2024 Northwest 62nd Street
Seattle Washington 98107(US)

(72) Inventor: Urdal, David Lloyd
6826 55th Avenue Northeast
Seattle Washington 98115(US)

(74) Representative: Sheard, Andrew Gregory et al,
Kilburn & Strode 30, John Street
London WC1N 2DD(GB)

(54) **Human G-CSF protein expression.**

(57) Human granulocyte colony stimulating factor (hG-CSF) and related mutant cDNAs and proteins are disclosed, characterized by molecular modifications increasing expression in microbial systems. Related aspects concern recombinant yeast and bacterial expression vectors for producing the proteins in high yield, and processes for making the proteins using microbial expression systems.

EP 0 243 153 A2

Croydon Printing Company Ltd

## TITLE

Human G-CSF Protein Expression

## BACKGROUND OF THE INVENTION

The present invention relates generally to lymphokines, and particularly to growth factors which induce differentiation and proliferation of human hematopoietic progenitor cells.

Human blood cells derive from pluripotent hematopoietic stem cells originating in the bone marrow. These stem cells provide progenitor cells for erythrocytes, polymorphonuclear granulocytes, monocytes, B and T lymphocytes and platelets. Polymorphonuclear granulocytes, characterized by lobulated nuclei and abundant pseudoplasmic granules, differentiate into neutrophils, eosinophils and basophils. Monocytes and lymphocytes differentiate along particular pathways to transform into macrophages which are indistinguishable from typical tissue macrophages.

The differentiation and proliferation of hematopoietic cells is regulated by secreted glycoproteins collectively referred to as colony stimulating factors (CSFs). In murine and human systems, these proteins include granulocyte-macrophage colony stimulating factor (GM-CSF), which promotes granulocyte and macrophage production from normal bone marrow, and which also appears to regulate the activity of mature, differentiated granulocytes and macrophages. Other CSFs include macrophage CSF (M-CSF or CSF-1), which induces the selective proliferation of macrophages, and burst promoting activity (BPA), which induces development of erythroid cell progenitors into hemoglobin-containing cells. An additional murine CSF, known variously as IL-3 or multi-CSF, stimulates the development of numerous cell types of hematopoietic lineage. Gibbon and human homologues of murine IL-3 have been reported by Yang et al., Cell 47:3 (1986).

Granulocyte-specific colony stimulating factors (G-CSFs) induce differentiation and expansion of granulocyte-committed progenitor cells from multipotent hematopoietic stem cells. When administered to mammals, G-CSFs promote a dramatic increase in circulating granulocyte populations.

Murine and human granulocyte colony stimulating factors have been isolated and partially characterized. Murine G-CSF was first purified from endotoxin-stimulated mouse lung conditioned medium, as described by Nicola et al., J. Biol. Chem. 258:9017 (1983). The murine protein induced terminal differentiation of the WEHI-3B murine myelomonocytic leukemia cell line, and stimulated neutrophilic granulocyte colonies in semisolid agar. At low concentrations, murine G-CSF stimulated granulocyte colony precursors exclusively; at higher concentrations, it stimulated mixed granulocyte/macrophage colony formation. Isolation and sequencing of a cDNA encoding murine G-CSF was reported by Tsuchiya et al., Proc. Natl. Acad. Sci. USA 83:7633 (1986).

Welte et al., Proc. Natl. Acad. Sci. USA 82:1526 (1983) reported partial purification and preliminary characterization of a CSF found in supernatants of the human bladder carcinoma cell line HBT 5637. This factor, termed "pluripotent hematopoietic colony-stimulating factor", supported growth of human mixed colonies, granulocyte-macrophage colonies, and early erythroid colonies. This CSF also induced terminal differentiation of the WEHI-3B cell line. An apparent molecular weight of about 18,000 was reported.

Nomura et al., EMBO Journal 5:871 (1986), established a human oral cavity carcinoma cell line (CHU-2), which constitutively produced a granulocyte-lineage specific CSF. This 19,000 dalton protein exhibited an assay profile largely coincident with that of murine G-CSF. Nagata et al., Nature 319:415 (1986) isolated two cDNAs for the CHU-2 G-CSF protein which differed with respect to a three amino acid insert. When expressed in mammalian COS cells, both cDNAs provided proteins with G-CSF activity. Sequencing of the cDNAs and a genomic G-CSF clone suggested that the sequence containing the insert was the product of an alternative mRNA splice. The protein lacking the insert had greater biological activity.

Souza et al., Science 232:61 (1986) purified G-CSF from HBT 5637 cell supernatants, and isolated a cDNA from an HBT 5637 library which had a sequence homologous to the CHU-2 G-CSF clone lacking the three codon insert. Souza et al. cloned and expressed the HBT 5637 G-CSF sequence in *Escherichia coli* to provide active protein.

Due to potential clinical utility as a stimulator of granulocytic cell precursors, there is considerable interest in G-CSF in the hematology and oncology communities. Therapeutic compositions with G-CSF activity could be employed to potentiate immune responsiveness to infectious pathogens, or to assist in reconstituting normal blood cell populations following radiation- or chemotherapy-induced hematopoietic cell suppression. G-CSF may also find application in the treatment of certain leukemias, due to its ability to cause differentiation of certain neoplastic cells of hematopoietic lineage. The present invention concerns novel mutant G-CSF proteins which are expressed and secreted in high yields by yeast cells.

## SUMMARY OF THE INVENTION

The present invention provides purified human granulocyte colony stimulating factor (hG-CSF) as a homogeneous protein, processes for its production from cell cultures, and cDNA's which encode hG-CSF. In related aspects, the present invention is directed to human G-CSF (hG-CSF) muteins having A/T enriched 3' coding regions and/or inactivated yeast KEX2 protease processing sites. These mutant proteins are expressed in very high yield in yeast and bacterial expression systems. The invention also concerns recombinant expression vectors comprising nucleotide sequences encoding the muteins, related microbial expression systems comprising appropriate host organisms transformed with the foregoing recombinant expression vectors, and processes for making the muteins using the microbial expression systems.

## BRIEF DESCRIPTION OF THE DRAWINGS

FIG. 1 compares the nucleotide sequences and corresponding amino acid sequences of native human G-CSF and hG-CSF[$Lys^{22}$], a preferred mutein having an inactivated KEX2 site and other nucleotide changes resulting in enhanced expression. FIG. 1 also indicates the position of preferred locations for elimination or addition of cysteine residues to stabilize expressed protein.

FIG. 2 is a schematic representation of the construction of plasmid pBC102·K22, which comprises a cDNA sequence encoding hG-CSF[$Lys^{22}$]. This plasmid, resident in *E. coli* strain RR1, has been deposited with the American Type Culture Collection (ATCC) under accession number 67,255.

## DETAILS OF THE INVENTION

Crude preparations of hG-CSF are prepared by initially culturing malignant, neoplastic hematopoietic growth factor producing cells *in vitro* in a serum containing medium supplemented with various additives. Conditioned medium is prepared from the cells by first growing the cells to confluence and then removing the medium from the cells for replacement with fresh culture medium containing serum. After an appropriate culture duration, the medium is harvested and processed to purify the hematopoietic growth factor into a more concentrated form.

Various cells and cell lines can be employed in the production of crude hematopoietic growth factor, including various malignant neoplastic cell lines, for example carcinoma, sarcoma, lymphoma, leukemia, melanoma and myeloma cell lines. An exemplary cell line is the human bladder carcinoma cell line HBT 5637, which is publicly available from the American Type Culture Collection, 12301 Parklawn Drive, Rockville, MD 20852, USA, under accession number HTB-9.

Suitable culture media for production of crude hG-CSF include such commercially available media as Roswell Park Memorial Institute (RPMI)-1600 medium, Dulbecco's Modified Eagle Medium (DMEM) and Click's Medium. Additives such as the antibiotics penicillin, streptomycin, and gentamycin can be added to media. Additional additives may include various buffers, such as HEPES buffer (N-2-hydroxyethylpiperazine-N-2-ethane-sulfonic acid). Further additives may include L-glutamine, $NaHCO_3$, 2-mercaptoethanol and various serums, including fetal calf serum (FCS) or normal human serum. Preferably, cultures are maintained at 35-38°C in a humidified atmosphere of 5-10% $CO_2$ in air, at pH 7.0-7.4.

Various assays can be employed to detect granulocyte-specific hematopoietic growth factors. A first assay, referred to herein as a "bone marrow assay" measures capacity of a sample to induce proliferation of bone marrow cells obtained from both human or murine sources. Cells are stained to visualize the cell types present in induced colonies. hG-CSF induces formation of mature granulocytes and neutrophils from both human and murine bone marrow.

A second assay measures capacity of a sample to cause proliferation of IL-3 dependent cells such as 32D cells, FDC-P2 cells, FDC-P1 cells and FDC-P2-1d cells, all of which are widely available. 32D cells are derived from a retrovirus-infected C3H-HeJ mouse bone marrow culture, as described by Greenburger, et al., Fed. Proc. 42:2762 (1983). hG-CSF scores positively in IL-3 dependent cell proliferation assays, indicating that the activity of the factor is not restricted to simply granulocytes or macrophages.

A third assay for granulocyte-specific CSF involves measuring the capacity of a sample to cause leukemic cells to differentiate into mature leukocytes. Exemplary cell lines for this purpose include the murine myelomonocytic WEHI-3 (ATCC TIB-68) cells and the human myelocytic leukemia cell line, HL60, from the human erythroleukemia, KG1. This particular assay procedure of the present invention has been conducted with WEHI-3B D+ cells. Nicola and Metcalf, J. Cell.

Phys. 109:253 (1981) describe use of VEHI-3B D+ cells to detect the presence of G-CSF.

Crude hG-CSF produced in supernatants of HBT 5637 or other suitable cells is initially purified by ammonium sulfate precipitation, followed by gel column chromatography and then by multiple HPLC procedures. The progress of purification can be monitored by gel electrophoresis followed by silver staining.

Preferred gel filtration media include agarose, Sephadex, polyacrylamide, (e.g., Bio-Gel), or mixtures of the foregoing such as AcA-44 ultragel or AcA-54 ultragel. AcA-54 ultragel is most preferred. hG-CSF derived from crude culture supernatants eluted from gel filtration columns in a molecular weight range from 15,000-30,000 daltons.

Gel filtration fractions containing hG-CSF activity are pooled for preparative HPLC procedures, preferably employing a reverse phase, octadecyl bonded silica column having a pore size of at least 15-20 microns. Such columns include the Radiopak and Porasil lines of columns commercially available from Waters Associates (Milford, ME, USA). Prior to applying the hematopoietic growth factor to the column, fractions are diluted and the column equilibrated with a buffer comprising trifluoroacetic acid (TFA), heptafluorobutyric acid (HFBA) or acetic acid. hG-CSF can be eluted from RP-HPLC columns using a linear gradient of acetonitrile or an N-propanol buffer solution in TFA, HFBA or acetic acid. If acetonitrile is used as eluant, a preferred gradient is 0-100% (V/V) of acetonitrile in TFA, applied to the column at a rate of approximately 1% acetonitrile per minute. If the eluant is N-propanol, a preferred gradient is 60% (V/V) n-propanol in 0.9 M acetic acid and 0.2 M pyridine (pH 4.0). A preferred gradient range for this buffer eluant is from 0-100% and, more preferably, from 0-84%.

If sufficient protein purification is not achieved by the initial HPLC procedure, it can be repeated, using similar or different elution buffers. As detailed below, a single HPLC step using C14 bonded phase and elution with aqueous TFA did not sufficiently purify hG-CSF from cell media to provide a single specific protein band by gel electophoresis. However, after a second HPLC treatment employing the same column but with an N-propanol/acetic acid/pyridine eluant, a distinct band at about 19,000 daltons was recovered which corresponded to hematopoietic growth factor activity.

To achieve homogeneity, a third HPLC step was undertaken, employing a C18 column equilibrated with TFA, which was eluted with an acetonitrile gradient. Following this step, homogeneous hematopoietic growth factor activity was observed in a single fraction having a molecular weight of approximately 19,000 daltons. The hematopoietic growth factor (hG-CSF) was found to have a specific activity of about $10^6$ units per microgram (U/μg) in the 32D cell line proliferation assay and $10^6$ colony forming units per microgram (CFU/μg) in the murine bone marrow colony forming assay.

The hematopoietic growth factor activity was found to be stable for at least six months when stored at 4°C in organic buffers used in HPLC fractionation, supra, (TFA/acetonitrile) or (pyridine/acetate/propanol).

Samples of the homogeneous protein were analyzed for amino acid composition and sequence using an automated sequencer employing either ninhydrin or gas phase detection. The first 25 residues of the amino terminal portion of the hematopoietic growth factor of the present invention are composed of the following sequence:
Thr-Pro-Leu-Gly-Pro-Ala-Ser-Ser-Leu-Pro-Gln-Ser-Phe-His-Cys-Lys-Asn-Leu-Glu-Gln-Val-Arg-Ile-Lys-Ile.

A DNA segment encoding native human G-CSF was isolated from a cDNA library prepared by reverse transcription of polyadenylated RNA isolated from a human bladder carcinoma cell line, HBT 5637 (ATCC HBT-9). Synthetic oligonucleotide probes having sequence homology to N-terminal, C-terminal, and 3′ non-coding regions of the native human mRNA sequence were employed to screen the library by conventional DNA hybridization techniques. DNA was isolated from those clones which hybridized to the probes and analyzed by restriction endonuclease cleavage, agarose gel electrophoresis, and additional hybridization experiments ("Southern blots") involving the electrophoresed fragments. After isolating a single clone which hybridized to each of the probes, the hybridizing segment bearing the hG-CSF gene was then subcloned and sequenced by conventional techniques. A part of this fragment, encoding amino acids 43-174 of mature hG-CSF, was identified and cleaved from the remainder of the fragment with a particular restriction endonuclease. This DNA segment was ligated to a synthetic N-terminal sequence comprising a 3 codon deletion and added internal restriction sites. This construct was then altered by site-specific mutagenesis procedures to provide a mutant analog sequence in which the codon specifying $Arg^{22}$ (AGA) was replaced by a codon specifying Lys (AAA).

The mutant or altered sequence was then inserted into a yeast expression vector under control of a particular promoter. The vector was used to transform an appropriate yeast expression strain, which was grown in culture under conditions promoting derepression of the yeast promoter. The resulting yeast-conditioned culture supernatant was assayed to confirm enhanced expression of hG-CSF[$Lys^{22}$].

## Definitions

"Human granulocyte colony stimulating factor" and "hG-CSF" refer to a human endogenous secretory protein which selectively induces development of granulocyte-committed progenitors from multipotent hematopoietic cells.

"Mutant amino acid sequence" refers to a polypeptide encoded by a nucleotide sequence intentionally made variant from a native sequence. "Mutant protein" or "mutein" means a protein comprising a mutant amino acid sequence. "Substantially homologous," which can refer both to nucleic acid and amino acid sequences, means that a particular subject sequence, for example, a mutant sequence, varies from a reference sequence by one or more substitutions, deletions, or additions, the net effect of which do not result in an adverse functional dissimilarity between reference and subject sequences. For purposes of the present invention, sequences having greater than 80 percent homology, equivalent biological specific activity, and equivalent expression characteristics are considered substantially homologous. Sequences having lesser degrees of homology, comparable bioactivity, and equivalent expression characteristics are considered equivalents. "Native sequence" refers to an amino acid or nucleic acid sequence which is identical to a wild-type or native form of a gene or protein. "KEX2 protease recognition site" is defined below. The term "inactivate", as used in defining the present invention, means to alter a selected KEX2 protease recognition site to retard or prevent cleavage by the KEX2 protease of Saccharomyces cerevisiae.

"Recombinant," as used herein, means that a protein is derived from recombinant microbial (e.g., bacterial or fungal) expression systems. "Crude culture supernatant" refers to media withdrawn from yeast or bacterial cultures which has not been subjected to concentration or purification procedures.

"DNA segment" refers to a DNA polymer, in the form of a separate fragment or as a component of a larger DNA construct, which has been derived from DNA isolated at least once in substantially pure form, i.e., in a quantity or concentration enabling identification, manipulation, and recovery of the segment and its component nucleotide sequences by standard biochemical methods, for example, using a cloning vector. "Nucleotide sequence" refers to a heteropolymer of deoxyribonucleotides. "Recombinant expression vector" refers to a plasmid comprising a transcriptional unit comprising an assembly of (1) a genetic element or elements having a regulatory role in gene expression, for example, promoters or enhancers, and (2) a structural or coding sequence which is transcribed into mRNA and translated into protein. Preferably, the transcriptional unit includes a leader sequence enabling extracellular secretion of translated protein by a host cell. "Recombinant expression system" means a combination of an expression vector and a suitable host microorganism. "Substantially pure" means at least 75%, 80%, 90%, 95% or 99% by weight pure in increasing order of preference.

## Assays for hG-CSF Biological Activity

Assays used to measure hG-CSF biological activity are described below.

### 1. Human and Murine Bone Marrow Colony Assays

In this assay, 50 µl samples are plated in appropriate wells in log-2 dilution series. A 1.4% agar suspension is prepared by heating in a boiling water bath, and then held at 40°C prior to use. An incubation medium is prepared by mixing seven parts nutrient medium [α-minimum essential medium (αMEM) supplemented with vitamins, 28.5% fetal bovine serum (FBS), $0.7 \times 10^{4}$ M 2-mercaptoethanol, 0.12 mg/ml asparagine, 0.7 mg/ml glutamine, 150 U/ml penicillin G, and 150 U/ml streptomycin] and three parts agar suspension, and held at 37°C. Percoll treated bone marrow cells are warmed to 37°C and added to the incubation medium to provide a final concentration of approximately $1 \times 10^{5}$ cells/ml. The resulting mixture is kept at 37°C while dispensing 250 µl aliquots into each well. Plates are held at about 23°C until the agar solidifies, then incubated at 37°C in plastic boxes containing distilled water to prevent desiccation.

Colonies having 50 or more cells each are counted on days 7 or 10 and 14. Earlier counts are better for granulocyte colonies, while later counts are better for macrophage and mixed colonies. In each assay, several wells are plated without hematopoietic growth factor samples to obtain a background colony count. hG-CSF activity, expressed in colony forming units per milliliter ("CFU/ml"), is defined as that sample dilution providing one-half of the maximum colonies formed by $1 \times 10^{5}$ bone marrow cells, multiplied by the number of colonies observed in the half maximal case.

Cell types in colonies are determined by staining individual cells with a stain consisting of 0.6% Orcein and 60% acetic acid. hG-CSF samples can also be assayed using murine bone marrow cells in a protocol substantially similar to that described above. In the murine assay, horse serum is used in place of FBS, marrow cells are plated at a concentration of 5 x $10^4$ cells per ml incubation medium, and colonies are counted on days 4 or 5 and 7.

2. WEHI Differentiation Assay

This assay determines the capacity of a sample to induce terminal differentiation of a murine myelomonocytic leukemic cell line, WEHI-3B D+. This cell line has been widely distributed among researchers and is available from numerous sources, including Immunex Corporation, 51 University Street, Seattle, Washington, 98101. In the presence of phorbol myristate acetate (PMA), granulocytes resulting from hG-CSF-induced differentiation of WEHI-3B D+ cells convert nitro-blue tetrazolium (NBT) intracellularly from yellow to blue.

WEHI-3B D+ cells are maintained in culture in α-MEM supplemented with 15% FBS, 2 x $10^{-5}$ M 2-mercaptoethanol, 50 U/ml penicillin, 50 µg/ml streptomycin and 300 µg/ml fresh L-glutamine ("complete α-MEM"), and are subcultured every 3-4 days. The cells are counted and seeded into tissue culture flasks in 25-50 ml of medium at a density of approximately 1 x $10^5$ cells/ml. The cells double in 15% FBS every 1-2 days.

In the assay, 50 µg of complete α-MEM are dispensed into each well of a 96-well plate. Duplicate twofold serial dilutions of each sample are prepared, and 60 µl of a WEHI-3B D+ cell suspension (1 x $10^5$ cells/ml) is dispersed into each well. Plates are incubated at 37°C in the presence of 7% $O_2$, 10% $CO_2$ at high humidity. After 72 hours, 10 µl of a fresh NBT-PMA solution (2 mg/ml NBT and 40 µg/ml PMA in PBS) is dispensed into the first row of the duplicate rows and 100 µl of NBT (2 mg/ml in PBS) is dispensed into the second row. After an additional 1-2 hours at 37°C, 25 µl of 40 mM HCl in isopropanol is added to each well. Absorbance at 570 nM is then determined. Dye conversion is proportional to the number of granulocytes present, which, in turn, is proportional to hG-CSF activity. Units of activity in the sample are equal to the reciprocal of the dilution providing one-half maximum NBT conversion.

3. 32D Cell Proliferation Assay

This assay ascertains the capacity of a sample to induce proliferation of 32D cells, which are derived from retrovirus-infected C3H-HeJ mouse bone marrow cells. 32D cells have been widely distributed among researchers and are commonly available from many sources, including Immunex Corporation.

32D cells are routinely cultured in RPMI-1640 containing 5% (v/v) WEHI-3 conditioned medium, prepared from a 48 hour culture of WEHI-3 cells (1-5 x $10^6$ cells/ml) in RPMI-1640 medium supplemented with horse serum (5-20%, v/v), penicillin (50 U/ml), streptomycin (50 µg/ml), fresh L-glutamine (300 µg/ml) and 2-mercaptoethanol (25 x $10^5$ M). Cells are subcultured every 2-3 days. Cells are counted and seeded into culture flasks in 25-50 ml of medium at about 1-4 x $10^4$ cells/ml. The cells double every 12-24 hours at a relatively constant growth rate, and are maintained at 35-38°C in a humidified atmosphere of 5% $CO_2$ in air. To assay hG-CSF activity, 32D cells are plated in 96-well flat bottom microtiter plates (2 x $10^4$ cells/well) in a volume of 200 µl, together with a twofold dilution series of samples to be tested, and then incubated for 24-48 hours at 37°C in a humidified atmosphere of 5% $CO_2$ in air. Cells are pulsed for approximately 16 hours with 0.5 uCi of tritiated thymidine ($^3$H-Tdr, New England Nuclear, Boston, MA, 20 Ci/mM specific activity), harvested on glass fiber filter strips, and then counted by liquid scintillation counting. 32D cells incubated with hG-CSF incorporate radiolabel in a dose-dependent manner. 32D cells cultured in the absence of hematopoietic growth factors incorporate only background levels of $^3$H-Tdr. Units of activity are calculated as the reciprocal dilution of a sample providing 50% maximal thymidine incorporation.

## The hG-CSF Native Sequence

The nucleotide and deduced amino acid sequences of a DNA segment containing the hG-CSF gene isolated as described below is set forth in FIG. 1. In FIG. 1, nucleotides are numbered beginning with the ACC codon corresponding to the N-terminal threonine of the mature native protein. Similarly, amino acids are numbered from this threonine residue. The native polypeptide includes a leader sequence which is cleaved upon secretion to provide mature protein. The native protein includes an Arg-Lys pairing at position 22, which is susceptible to cleavage by the KEX2 protease of S. cerevisiae.

In accordance with the present invention, site-specific mutagenesis procedures are employed to inactivate KEX2 protease processing sites by deleting, adding, or substituting residues to alter Arg-Arg, Arg-Lys, or Lys-Arg pairs to eliminate the occurrence of these adjacent basic residues. Lys-Lys pairings are considerably less susceptible to KEX2 cleavage, and conversion of Arg-Lys or Lys-Arg to Lys-Lys represents a conservative and preferred approach to inactivating KEX2 sites. The resulting muteins are less susceptible to cleavage by the KEX2 protease at locations other than the yeast α-factor leader sequence, where cleavage upon secretion is intended.

The native hG-CSF sequence also includes an Arg-Arg pairing at residues 146-147. Muteins wherein this KEX2 site is inactivated are also within the scope of the present invention.

FIG. 1 also depicts a synthetic sequence of nucleotides 1-145 which was spliced to a C-terminal fragment of the native hG-CSF gene to provide a preferred mutant nucleotide sequence. This synthetic sequence varies from the native sequence by an "A/T enriched" N-terminal region wherein the proportion of deoxyadenosine and deoxythymidine residues is increased from 36% to 57%, promoting efficient expression in E. coli. In one embodiment of the present invention, this mutant sequence is linked to a yeast α-factor leader sequence, optionally via an N-terminal fusion construct comprising a nucleotide encoding the peptide Asp-Tyr-Lys-Asp-Asp-Asp-Asp-Lys (DYKDDDDK). The latter sequence is highly antigenic and provides an epitope reversibly bound by specific monoclonal antibody. This sequence is also specifically cleaved by bovine mucosal enterokinase at the residue immediately following the Asp-Lys pairing. Fusion proteins capped with this peptide are also resistant to intracellular degradation prior to secretion.

Construction of Analog Sequences and Muteins

The characteristic feature of the mutant proteins and nucleotide sequences of this invention is the conversion of the native Arg-Lys pairing at residues 22 and 23 to a configuration less susceptible to KEX2 protease cleavage. However, numerous DNA constructions including all or part of the nucleotide sequences depicted in FIG. 1, in conjunction with oligonucleotide cassettes, comprising additional useful restriction sites, can be prepared as a matter of convenience. Expression vectors and systems comprising such constructions, as well as hG-CSF muteins produced by such systems, are within the scope of the present invention.

Mutations can be introduced at particular loci by synthesizing oligonucleotides containing a mutant sequence, flanked by restriction sites enabling ligation to fragments of the native sequence. Following ligation, the resulting reconstructed sequence encodes a mutein having the desired amino acid insertion, substitution, or deletion.

Alternatively, oligonucleotide-directed site-specific mutagenesis procedures can be employed to provide an altered gene having particular codons altered according to the substitution, deletion, or insertion required. Walder et al., Gene 42:133 (1986); Bauer et al., Gene 37:73 (1985); Craik, Biotechniques, January 1985, 12-19; Smith et al., Genetic Engineering: Principles and Methods (Plenum Press, 1981); and U. S. Patent 4,518,584 disclose suitable techniques, and are incorporated by reference herein.

For either approach, conventional techniques for oligonucleotide synthesis are suitable, for example, the triester synthesis procedures disclosed by Sood et al., Nucl. Acid Res. 4:2557 (1977) and Hirose et al., Tet. Lett. 28:2449 (1978).

In site-specific mutagenesis, a strand of the gene to be altered is cloned into an M13 single-stranded phage or other appropriate vector to provide single-stranded (ss) DNA comprising either the sense or antisense strand corresponding to the gene to be altered. This DNA is then hybridized to an oligonucleotide primer complementary to the sequence surrounding the codon to be altered, but comprising a codon (or an antisense codon complementary to such codon) specifying the new amino acid at the point where substitution is to be effected. If a deletion is desired, the primer will lack the particular codon specifying the amino acid to be deleted, while maintaining the correct reading frame. If an insertion is desired, the primer will include a new codon, at the appropriate location in the sequence, specifying the amino acid to be inserted. Preferably, the substitute codon, deleted codon, or inserted codon is located at or near the center of the oligonucleotide.

The size of the oligonucleotide primer employed is determined by the need to optimize stable, unique hybridization at the mutation site with the 5′ and 3′ extensions being of sufficient length to avoid editing of the mutation by exonucleases. Thus, oligonucleotides used in accordance with the present invention will usually contain from about 15 to about 25 bases. Oligonucleotides of greater size are not needed.

In a preferred approach (see Walder et al., supra), the resulting oligonucleotide/ss vector hybrid is directly transformed into yeast. Alternatively, a mutagenic primer is hybridized to a gapped duplex having a single-stranded template segment containing the gene to be altered. In the latter case, the primer is extended along the template strand by reaction with DNA polymerase I (Klenow fragment), T4 DNA polymerase, or other suitable DNA polymerase, providing a resulting double stranded DNA which is circularized and used to transfect a suitable host strain. In both cases, replication of the heteroduplex by the host provides progeny of both strands. In *E. coli*, transfected cells are plated to provide colonies, which are screened using a labelled oligonucleotide corresponding to that used in the mutagenesis procedure. If yeast are transformed directly, transformants are pooled, DNA isolated and transformed into E. coli. The resulting colonies are screened by hybridization. Conditions are employed which result in preferential hybridization of the primer to the mutated DNA but not to the progeny of the parent strand. DNA containing the mutated gene is then isolated and spliced into a suitable expression vector, and the vector used to transform a host strain. The host strain is then grown in culture to provide the analog protein.

Protein Expression in Recombinant Yeast Systems

Yeast systems are preferred for expression of the recombinant analog proteins of this invention, e.g., the hG-CSF[$Lys^{22}$] mutein. An exemplary expression vector is pBC102·K22 (ATCC 67,255) which contains DNA sequences from pBR322 for selection and replication in E. coli ($Ap^r$ gene and origin of replication) and yeast DNA sequences including a glucose-repressible alcohol dehydrogenase 2 (ADH2) promoter. The ADH2 promoter has been described by Russell et al., J. Biol. Chem. 258:2674 (1982) and Beier et al., Nature 300:724 (1982). Plasmid pBC102·K22 also includes a Trp1 gene as a selectable marker and the yeast 2μ origin of replication. Adjacent to the promoter is the yeast α-factor leader sequence enabling secretion of heterologous proteins from a yeast host. The α-factor leader sequence is modified to contain, near its 3′ end, an Asp718 (KpnI and Asp718 are isoschisomers) restriction site to facilitate fusion of this sequence to foreign genes, and the DYKDDDDK sequence described above. To allow efficient processing of secreted protein, as described by Brake et al., Proc. Natl. Acad. Sci. USA 81:4642 (1984), a sequence coding for the Glu-Ala-Glu-Ala amino acids was omitted. Details regarding construction of this plasmid are provided below.

Alternative expression vectors are yeast vectors which comprise an α-factor promoter, for example pYαHuGM (ATCC 53157), which

bears the wild-type human GM-CSF gene. Others are known to those skilled in the art. The construction of pYαHuGM is described in EP-A-0183350, the disclosure of which is incorporated by reference herein.

The choice of appropriate yeast strains for transformation will be determined by the nature of the selectable markers and other features of the vector. Appropriate S. cerevisiae strains for transformation by pBC102·K22 or pYαHuGM, and various constructions derived from those vectors, include strains X2181-1B, available from the Yeast Genetic Stock Center, Berkeley, CA, USA [see below], having the genotype α trp1 gal1 ade1 his2; J17 (ATCC 52683; α his2 ade1 trp1 met14 ura3); and IL166-5B (ATCC 46183; α his1 trp1). A particularly preferred expression strain, XV2181, is a diploid formed by mating two haploid strains, X2181-1B, available from the Yeast Genetic Stock Center, Department of Biophysics and Medical Physics, University of California, Berkeley, CA 94702, USA; and XV617-1-3B, available from the Department of Genetics, University of Washington, Seattle, WA 98105, USA, or Immunex Corporation, 51 University Street, Seattle, WA 98101, USA. A suitable transformation protocol is that described by Hinnen, et al., Proc. Natl. Acad. Sci. USA 75:1929 (1978), selecting for $Trp^+$ transformants in a selective medium consisting of 0.67% yeast nitrogen base, 0.5% casamino acids, 2% glucose, 10 µg/ml adenine and 20 µg/ml uracil.

Host strains comprising pBC102·K22 or other constructions comprising the ADH2 or α-factor promoters are grown for expression in a rich medium consisting of 1% yeast extract, 2% peptone, and 1% glucose supplemented with 80 µg/ml adenine and 80 µg/ml uracil. Derepression of the ADH2 promoter occurs upon exhaustion of medium glucose.

Example 1: Purification of hG-CSF from HBT 5637 Conditioned Medium

HBT 5637 adherent human bladder carcinoma cells (ATCC No. HTB9) were grown to confluence in RPMI-1640 medium supplemented with 10% FCS. Once the cells were at confluence, medium from the cells was decanted and replaced with fresh RPMI-1640 medium containing 0.1% FCS. The cells were then cultured for approximately 72 hours in a humidified atmosphere of 5% $CO_2$ in air. Thereafter, the medium was harvested and centrifuged at 2000 Xg to remove floating or dying cells.

HBT 5637 conditioned medium as prepared above was initially concentrated by ammonium sulfate precipitation. The medium was brought to 80% saturation by adding solid ammonium sulfate at 4°C with gentle stirring. The ammonium sulfate was added over an extended time period of approximately 12 hours. At 80% saturation, gentle stirring was continued for an additional 4 to 8 hours and the resulting mixture centrifuged at 10,000 Xg for 30 minutes. The final protein pellet was then resuspended in PBS (phosphate buffered saline), pH 7.2, and dialyzed against multiple volumes of PBS, pH 7.2 for 12 to 24 hours at 4°C. The dialyzed concentrate was further purified by gel-filtration chromatography with a 2.5 x 90 cm AcA-54 ultragel column (an acrylamide-agarose ultragel having 100 to 10 k Dalton fractionation, LKB, Bromma, Sweden). Prior to loading, the column was equilibrated with PBS buffer. Fractions were eluted from the column with PBS containing 20 μg/ml gentamycin and assayed for biological activity. Active fractions eluted from the column at an apparent molecular weight of 15,000-30,000 daltons.

Active fractions from the gel chromatography step were pooled and directly applied to a Waters Radiopak HPLC column (Waters Associates), 8 mm x 10 cm, packed with Vydac C4 resin, 15-20 micron particle size, and equilibrated with 0.1% TFA (v/v). Total protein applied was generally about 4-40 mg in a volume of about 20-300 ml. The loaded column was washed with 0.1% TFA (v/v) until absorbence at 214 nm reached baseline values, then eluted with a linear gradient of 0-100% acetonitrile in 0.1% TFA (v/v) at 1% acetonitrile per minute. One minute fractions were collected and assayed for biological activity. Fractions containing activity were pooled, diluted with 10 ml of buffer A (0.9 M acetic acid, 0.2 M, pyridine, pH 4.0) and reapplied to the Radiopak HPLC column, previously equilibrated with buffer A. The column was washed with buffer A to remove non-bound components, then eluted with a 0-84% gradient of buffer B (60% N-propanol in 0.9 M acetic acid, 0.2 M pyridine, pH 4.0). An initial 0-20% buffer B gradient was applied to the column over a period of 10 minutes and then a second gradient of 20-84% buffer B was applied over 110 minutes. The column was pumped at a rate of 1 ml/min. during which time 1.5 minute fractions were collected and assayed. SDS-PAGE analysis of the active fractions indicated a protein band of approximately 19,000 MW.

Fractions containing activity were pooled, diluted with 10 ml of 0.1% (v/v) aqueous TFA, and applied to a Vydac C18 column (Waters Associates, 3.9 mm x 30 cm, 10 micron particle size) equilibrated with 0.1% (v/v) aqueous TFA. The column was eluted with a linear gradient of 0-100% (v/v) acetonitrile in 0.1% TFA at a rate of 1% per minute (100 ml total). One minute fractions were collected and assayed. Homogeneity of the protein component of the active fraction was confirmed by SDS-PAGE and silver staining which yielded a single band at approximately 19,000 daltons. The specific activity of this homogeneous material was determined to be approximately $10^6$ U/µg by the 32D assay and $10^6$ CFU/µg by murine and human bone marrow colony assays.

The protein sample purified by the foregoing HPLC steps was sequenced using an automated amino terminal Edman degradation using an Applied Biosystem Model 470 Approaching Sequencer. In a first sequencing attempt using approximately 310 picomole of protein, 85% of the sequence could be assigned to a single protein. A second confirming run, using approximately 500 picomole of material, was then made. The resulting N-terminal sequence was Thr-Pro-Leu-Gly-Pro-Ala-Ser-Ser-Leu-Pro-Glu-Ser-Phe-His-Cys-Lys-Asn-Leu-Glu-Gln-Val-Arg-Ile-Lys-Ile.

Example 2: Isolation of cDNA encoding hG-CSF, Site-Specific Mutagenesis, and Yeast Expression of Active Mutant Protein

Synthetic oligonucleotides, designated A, B, and C, were constructed with sequences complementary to three selected sequences of the human G-CSF gene. Probe A, complementary to the sequence encoding part of the leader and first amino acid (Thr) of the mature G-CSF protein, had the sequence 5'-GGTGGCTTCCTGCACTGTCCA-3'. Probe B, which corresponded to the region encoding amino acids 36-42 of the mature protein, had the sequence 5'-GTAGGTGGCACACTCACTCAC-3'. Probe C, complementary to a 3' noncoding region of the native gene, had the sequence 5'-AACTCAGAAATGCAGGGAAGG-3'. The method of synthesis was a standard automated triester method substantially similar to that disclosed by Sood et al., Nucleic Acids Res. 4:2557 (1977) and Hirose et al., Tet. Lett. 28:2449 (1978). Following synthesis, the oligonucleotide was deblocked and purified by preparative gel electrophoresis. The oligonucleotides were terminally radiolabelled using $^{32}$P-ATP and T4 polynucleotide kinase by standard techniques, such as those disclosed by Maniatis et al., Molecular Cloning: A Laboratory Manual (Cold Spring Harbor Laboratory 1982), for use as screening probes.

A cDNA library was constructed by reverse transcription of polyadenylated mRNA isolated from total RNA extracted from a human bladder carcinoma cell line, HBT 5637 (ATCC HBT-9). The cDNA was rendered double-stranded using DNA polymerase I, blunt-ended with T4 DNA polymerase, methylated with EcoRI methylase to protect EcoRI cleavage sites within the cDNA, and ligated to EcoRI linkers. The resulting constructs were digested with EcoRI to remove all but one copy of the linkers at each end of the cDNA, and ligated to EcoRI-cut and dephosphorylated arms of bacteriophage λgt10 (Huynh et al., DNA Cloning: A Practical Approach, Glover, ed., IRL Press pp 49-78). The ligated DNA was packaged into phage particles to generate a library of 750,000 recombinants. 150,000 recombinants were plated on *E. coli* strain C600hfl$^-$ and screened by standard plaque hybridization techniques with the labeled oligonucleotide probes. Two clones were isolated from the library which hybridized to all three probes. These were plaque purified and used to prepare bacteriophage DNA which was digested with EcoRI. The digests were electrophoresed on an agarose gel, blotted onto nylon filters, and retested for hybridization. One clone was partially digested with EcoRI followed by preparative agarose gel electrophoresis, then subcloned into an EcoRI-cut derivative of the standard cloning vector pBR322 containing a polylinker having a unique EcoRI site, a BamH1 site and numerous other unique restriction sites. An exemplary vector of this type is described by Dente et al., Nucleic Acids Research 11:1645 (1983). Restriction mapping indicated the presence of restriction sites corresponding to those previously reported for human G-CSF.

Oligonucleotides were synthesized which encoded the C-terminal 5 amino acids of the yeast α-factor leader peptide, beginning with the KpnI site, and the N-terminal 46 amino acids of hG-CSF fused in-frame to the α-factor leader sequence. In FIG. 2, the synthetic N-terminal hG-CSF sequence is indicated as fragment A. This sequence included, at its C-terminal, an AvaI site and a cohesive end corresponding to a PstI restriction site, in addition to added internal HindIII and XbaI sites. This fragment lacked three codons corresponding to an internal Val-Ser-Glu sequence beginning at residue 36, and thus converted the +3 version of hG-CSF to the more active -3 version. See Nagata et al., Nature 319:415 (1986). The resulting 161 base pair KpnI-PstI fragment, constructed from 8 oligomers of approximately 40 nucleotides each, was cloned into a pBR322 derivative, pUC9, cut with KpnI and PstI.

Following transformation of a suitable *E. coli* strain with each of the plasmids described above, DNA was purified using standard techniques and digested as follows. The pUC9 vector containing the synthetic N-terminus (fragment A) was digested with KpnI and AvaI and a 145 base pair fragment recovered. The vector (pGEMBL in FIG. 2) containing the hG-CSF cDNA was first digested with StuI and then partially digested with AvaI. An approximate 310 base pair AvaI-StuI

fragment encoding the C-terminal amino acids 43-174 of hG-CSF was isolated from the partial digest. These fragments were ligated to a KpnI and StuI cut yeast expression vector, pBC10, which comprises the ADH2 promoter, α-factor leader sequence, yeast and E. coli origins of replication, yeast Trp1 gene, and E. coli $Ap^r$ gene. The resulting construct was designated pBC88.

pBC88 was digested with Asp718, which cleaves at KpnI sites, and ApaI, which cleaves at nucleotide 13 of the hG-CSF gene. The resulting large fragment was then ligated to the following oligonucleotide (oligo B in FIG. 2), which includes a part of the C-terminus of the α-factor leader sequence terminating in a KEX2 protease recognition site, an 8 codon sequence encoding an antigenic hydrophilic synthetic N-terminus (DYKDDDDK) capable of cleavage by bovine enterokinase, and a short sequence encoding the hG-CSF protein up to and including an ApaI cohesive end. The sequence of oligonucleotide B, as well as its corresponding amino acid sequence, are set forth below:

| | | | | | | | | | | | | | | | | | | |
|---|---|---|---|---|---|---|---|---|---|---|---|---|---|---|---|---|---|---|
| | | -12 | -11 | -10 | -9 | -8 | -7 | -6 | -5 | -4 | -3 | -2 | -1 | 1 | 2 | 3 | 4 | |
| | Pro | Cys | Asp | Lys | Arg | Asp | Tyr | Lys | Asp | Asp | Asp | Asp | Lys | Thr | Pro | Leu | Gly | |
| GTA | CCT | TTG | GAT | AAA | AGA | GAC | TAC | AAG | GAC | GAC | GAT | GAC | AAG | ACT | CCT | CTG | GGC | C |
| | GA | AAC | CTA | TTT | TCT | CTG | ATG | TTC | CTG | CTG | CTA | CTG | TTC | TGA | GGA | GAC | | |

The resulting construction was designated pBC90.

*In vitro* mutagenesis was conducted by a method similar to that described by Walder and Walder, Gene 42:133 (1986), but with the following differences. The expression vector pBC90 was modified to include a DNA fragment encoding the origin of replication for the single stranded bacteriophage f1, derived from the pEMBL vector described by Dente et al., Nucleic Acids Research 11:1645 (1983). This was achieved by cleaving pBC90 at a unique NruI site, and ligating the cleaved plasmid to a blunt-ended 514 bp RsaI fragment comprising the f1 origin. The resulting construct, designated pBC102, is capable of expressing G-CSF in yeast and also capable of generating single-stranded DNA when present in a suitable (male) strain of E. coli and superinfected with helper phage.

Single-stranded DNA was generated by transforming E. coli strain JM107 and superinfecting with helper phage IR1. Single-stranded DNA was isolated and annealed to the mutagenic oligonucleotide 5'-GAACAAGTTAAAAAAATCCAGC-3', (oligo D) which provides a codon switch substituting Lys for Arg at a position corresponding to residue 22 of mature native hG-CSF. Annealing and yeast transformation conditions were substantially similar to those disclosed by Walder and Walder, supra. Yeast transformants were selected by growth on medium lacking tryptophan, pooled, and DNA extracted substantially as described by Holm et al., Gene 42:169 (1986). This DNA, containing a mixture of wild-type and mutant plasmid DNA, was used to transform E. coli RR1 to ampicillin resistance. The resulting colonies were screened by hybridization to radiolabeled oligonucleotide D, using standard techniques. Plasmids comprising DNA encoding hG-CSF[$Lys^{22}$] were identified by the hybridization to radiolabeled oligonucleotide D under stringent conditions (e.g., 55°C, 6xSSC), and verified by nucleotide sequencing.

Plasmid DNA from an E. coli clone containing the hG-CSF[$Lys^{22}$] gene was then isolated and used to transform yeast strain XV2181 for expression of the altered gene product. Supernatants produced by the transformed yeast strain grown under conditions promoting derepression of the ADH2 promoter were then assayed by reaction with a mouse monoclonal antibody followed by horseradish peroxidase-conjugated goat anti-mouse antibody to detect the presence of the DYKDDDDK N-terminal peptide, and by bioassay for G-CSF activity. These experiments indicated approximately 5-fold greater expression of the mutein relative to parallel expression experiments involving the native protein.

Example 3: High Level Expression of hGSF in E. coli

Plasmid pBC88, assembled as described in Example 2, was digested with ApaI and StuI to provide an approximate 427 base pair fragment encoding the C-terminal amino acids 4-174 of mature hGCSF. This fragment is characterized by an "A/T enriched" N-terminal region, wherein the proportion of deoxyadenosine and deoxythymidine residues in the N-terminal 40 codons is increased relative to the native cDNA sequence.

A similar ApaI-StuI fragment was isolated from the cloning vector comprising the native hG-CSF sequence (pGEMBL in FIG. 2) to provide the region of the native cDNA sequence corresponding to the A/T-enriched version.

The native and synthetic fragments were each ligated to the following oligonucleotide E, which provides cohesive ClaI and ApaI terminals and codons encoding an N-terminal methionine residue and the first three amino acids of mature hG-CSF:

```
ClaI                            ApaI
  C GAT ACT ATG ACT CCT CTG GGC C
     TA TGA TAC TGA GGA GAC
                Met Thr Pro Leu Gly
```

The resulting fragments were ligated to a ClaI, SmaI-cut derivative of pGEM1 (Promega Biotec, Madison, Wisconsin, USA) with a DNA fragment containing a temperature-sensitive λ $P_L$ promoter, This fragment is substantially similar to that described in detail by Gourse et al., Proc. Natl. Acad. Sci. USA 82:1069 (1985). A related λ $P_L$ promoter sequence is present on the plasmid resident in *E. coli* strain JMB9, which has been deposited with the American Type Culture Collection (ATCC) under accession number ATCC 37092, and on plasmid pPL28, resident in *E. coli* RR1 and deposited as ATCC 53082.

This expression vector, designated pPL3, was further modified by replacement of the commercial multiple cloning site with the following KpnI-HindIII fragment:

```
     KpnI                            ClaI
GG GTA CCA AGT TCA CGT AAA AAG GGT ATC GAT ACT ATG GAC TAC AAA GAT
CC CAT GGT TCA AGT GCA TTT TTC CCA TAG CTA TGA TAC CTG ATG TTT CTA

     EcoRV   NcoI           SmaI
GAC GAT ATC CAT GGA ATT CGA GCT CGC CCG GGG ATC CTC TAG AGT CGA CCT
CTG CTA TAG GTA CCT TAA GCT CGA GCG GGC CCC TAG GAG ATC TCA GCT GGA

            HindIII
GCA GCC CAA GCT TGG
CGT CGG GTT CGA ACC
```

The three DNA fragments were ligated with T4 DNA ligase to provide intact expression plasmids identical except for the replacement of the first 128 base pairs of mature hG-CSF by a synthetic DNA segment. This segment has an A/T content enriched from 36% to 57% as described above and illustrated in FIG. 1. The native construct was designated pPL3/nG-CSF, while the A/T enriched version was designated pPL3/sG-CSF.

A third construction, designated pPL3/FLAG-sG-CSF, was assembled from the 427 base pair ApaI-StuI sG-CSF fragment fused to the following ClaI-ApaI oligonucleotide F. This fragment encodes an N-terminal methionine fused to a AspTyrLysAspAspAspAspLys (DYKDDDDK) hydrophobic leader sequence and the first three amino acids of mature hG-CSF. The DYKDDDDK sequence provides a highly antigenic leader capable of being bound by specific antibody and cleaved by purified bovine or porcine mucosal enterokinase, and is also capable of enhancing expression of fusion proteins.

```
ClaI                                                     ApaI
C GAT ACT ATG GAC TAC AAA GAT GAC GAT GAC AAA ACT CCT CTG GGC C
   TA TGA TAC CTG ATG TTT CTA CTG CTA CTG TTT TAC TGA GGA
          Met Asp Tyr Lys Asp Asp Asp Asp Lys Thr Pro Leu Gly
```

Finally, a fourth construction, designated pPL3/F-stop-sG-CSF, was assembled from the 427 base pair ApaI-StuI sG-CSF fragment fused to the following ClaI-ApaI oligonucleotide G, which encodes an N-terminal methionine fused to a AspTyrLysAspAspAsp (DYKDDD) hydrophobic leader sequence which had previously been noted to provide enhanced expression of fusion proteins, a stop codon, a second N-terminal methionine, and the first three amino acids of mature hG-CSF:

```
ClaI                                                                  ApaI
C GAT ACT ATG GAC TAC AAA GAT GAC GAT TAA CAT ATG ACT CCT CTG GGC C
   TA TGA TAC CTG ATG TTT CTA CTG CTA ATT GTA TAC TGA GGA GAC
          Met Asp Tyr Lys Asp Asp Asp End     Met Thr Pro Leu
```

Each of the foregoing expression vectors was then used to transform E. coli strain K802 (pRK248cIts; ATCC 33526) to tetracycline and ampicillin resistance. To test expression of proteins having G-CSF activity, the transformants were grown in L-broth without ampicillin at 30°C to an absorbance at 600 nm of about 0.4-0.5. The cultures were then shifted to 43°C for 1 hour to promote derepression of the $\lambda$-$P_L$ promoter. After three hours, cells from 1 mL culture were collected by centrifugation and the pellet extracted in 300 µl guanidine-HCl. The resulting extract was diluted 1:100 in phosphate-buffered saline, and assayed in the 32D murine bone marrow proliferation assay. The results are set forth in the following table:

| Construct | 32D Proliferation-Inducing Activity (U/mL culture) |
|---|---|
| pPL3 (no insert) | 0 |
| nG-CSF | 1,200 |
| sG-CSF | 130,000 |
| FstopG-CSF | 70,000 |
| Flag/sG-CSF | 1,600,000 |

Example 4: Alteration or Addition of Cysteine Residues

The native amino acid sequence of hG-CSF comprises five cysteine residues, located at positions 17, 36, 42, 64, and 74 of the mature hG-CSF molecule. In order to test whether alteration of the native sequence ($Arg^{22}$) by elimination of an odd-numbered cyteine would enhance expression by stabilizing the resulting 4-cysteine version, site-specific mutagenesis as previously described was employed to convert the codon encoding $Cys^{17}$ from TGT to AGT, thus specifying serine in place of cysteine. The mutagenic oligonucleotide employed corresponded to the native sequence from nucleotide 39 through nucleotide 60, with the exception of the substitution of deoxyadenosine for deoxythymidine at position 49. The resulting construct, assembled as a fusion protein having a DYKDDDDK N-terminal leader, was reinserted into a yeast expression vector substantially similar to pBC102·K22 except for identity of the target sequence. This vector, designated pBC122, was used to transform a suitable yeast host as previously described. The resulting transformants were then grown under conditions promoting derepression of the ADH2 promoter. Assay results are described below.

In a related experiment, $Phe^{83}$ was replaced by a cystein residue, to test the hypothesis that addition of a cysteine residue to provide a total of six cysteine residues would also enhance expression and recovery by stabilizing the molecule. Again, site-specific mutagenesis was employed to convert the codon encoding $Phe^{83}$ from TTC to TGC, thereby specifying cysteine. The mutagenic oligonucleotide employed corresponded to the native sequence from nucleotide 235 through nucleotide 261, with the exception of the substitution of deoxyguanosine for deoxythymidine at position 248. The resulting construct, incorporating a DYKDDDDK N-terminal leader, was reinserted into a yeast expression vector as described above. This vector, designated pBC121, was used to transform a suitable host, which was grown under conditions promoting derepression of the ADH2 promoter. Assay results are indicated in the following table.

Yeast culture supernatants from comparable fermenations were assayed at appropriate dilutions using the 32D murine bone marrow proliferation assay. In the table, pBC90 refers to a wild-type protein incorporating the DYKDDDDK leader, and pBC110 indicates a $Lys^{22}$ derivate of pBC102 also incorporating a DYKDDDDK leader. Immuno-dot blot assays using monoclonal antibody specific for the DYKDDDDK leader indicated qualitatively similar levels of antibody-reactive protein in each culture sample indicated below:

| Construct | 32D Proliferation-Inducing Activity in 1 ml Culture Supernatant |
|---|---|
| pBC90 | 439,667 |
| pBC110 | 820,492 |
| pBC121 (6-Cys) | 1,087,476 |
| pBC122 (4-Cys) | 1,400,864 |

## Therapeutic Applications

Hematopoietic growth factor (hG-CSF) may be employed to treat various stem and progenitor cell suppressions, such as those caused by chemotherapy and radiation therapy in cancer patients. Also, the factor may be used for the treatment of leukemia and various anemias. For such treatments, dosages in the range of 1 to 1 x $10^5$ µg per patient per treatment may be used. Administration may be by any suitable method, e.g., by injection in a pharmacological carrier, such as saline or saline mixed with human serum albumin.

CLAIMS FOR THE CONTRACTING STATES: BE, FR, DE, IT, LU, NL, SE, CH, LI and GB.

1. Purified or substantially pure human granulocyte colony stimulating factor (hG-CSF).

2. A human G-CSF (hG-CSF) mutein wherein at least one yeast KEX2 protease processing site has been inactivated.

3. An hG-CSF mutein having an amino acid sequence which is substantially homologous to the synthetic amino acid sequence of FIG 1.

4. An hG-CSF mutein wherein $Cys^{17}$ of the native sequence has been replaced by a non-Cys residue.

5. An hG-CSF mutein wherein $Phe^{83}$ of the native sequence has been replaced by a Cys residue.

6. A DNA segment encoding a human G-CSF protein, said segment characterized by increased deoxyadenosine and deoxythymidine content relative to the native hG-CSF sequence.

7. A DNA segment according to claim 6, having substantial homology to the synthetic DNA sequence of FIG 1.

8. A DNA segment encoding encoding a protein according to any one of claims 2 to 5.

9. A crude culture supernatant comprising a protein according to any one of claims 1 to 3 at a protein concentration of at least 10 mcg/ml.

10. A recombinant yeast expression vector comprising a DNA segment encoding hG-CSF or an hG-CSF mutein.

11. A recombinant microbial expression vector comprising a DNA segment according to any one of claims 6 to 8.

12. A recombinant expression vector which is or is derived from pBC102.K22 (ATCC 67,255).

13. A process for preparing hG-CSF or an h-GSF mutein, comprising culturing a host microorganism comprising an expression vector according to any one of claims 10 to 12 under conditions promoting expression.

CLAIMS FOR THE CONTRACTING STATES: AT and ES.

1. A process for the preparation of substantially pure or purified human granulocyte colony stimulating factor (hG-CSF), the process comprising applying a crude mixture containing hG-CSF to an HPLC column and eluting hG-CSF from the column.

2. A process for the preparation of an hG-CSF mutein, the process comprising coupling together by the formation of peptide bonds successive amino acid residues, wherein at least one amino acid residue at a yeast KEX2 processing site in the native sequence has been altered to render the site inactive.

3. A process for the preparation of an hG-CSF mutein having an amino acid sequence which is substantially homologous to the synthetic amino acid sequence shown in Figure 1, the process comprising coupling together by the formation of peptide bonds successive amino acid residues.

4. A process for the preparation of an hG-CSF mutein, the process comprising coupling together by the formation of peptide bonds successive amino acid residues, wherein $Cys^{17}$ of the native sequence has been replaced by a non-Cys amino acid residue.

5. A process for the preparation of an hG-CSF mutein, the process comprising coupling together by the formation of peptide bonds successive amino acid residues, wherein $Phe^{83}$ of the native sequence has been replaced by a Cys residue.

6. A process for the preparation of a DNA sequence encoding a human G-CSF protein, the segment having increased dA and dT content relative to the native hG-CSF sequence, the process comprising coupling together successive nucleotide residues.

7. A process as claimed in claim 6, wherein the DNA sequence has substantial homology to the DNA sequence of Figure 1.

8. A process for the preparation of a DNA sequence encoding a protein preparable by a process as claimed in any one of claims 2 to 5, the DNA sequence preparation process comprising coupling together successive nucleotide residues.

9. A process for the preparation of a crude culture supernatant comprising a protein preparable by a process as claimed in claim 1, 2 or 3, the supernatant preparation process comprising culturing cells capable of expressing the protein into the supernatant until the desired protein concentration in the supernatant is at least 10 mcg/ml.

10. A process for the preparation of a recombinant yeast expression vector, the process comprising ligating yeast expression vector DNA and a DNA segment encoding hG-CSF or an hG-CSF mutein.

11. A process for the preparation of a recombinant microbial expression vector, the process comprising ligating microbial expression vector DNA and a DNA segment preparable by a process as claimed in claim 6, 7 or 8.

12. A process as claimed in claim 10, wherein the expression vector is, or is derived from, pBC102.K22 (ATCC 67,255).

13. A process for preparing hG-CSF or an h-GSF mutein, comprising culturing a host microorganism comprising an expression vector produceable by a process according to any one of claims 10 to 12 under conditions promoting expression.

CLAIMS FOR THE CONTRACTING STATE: GR

1. A process for the preparation of substantially pure or purified human granulocyte colony stimulating factor (hG-CSF), the process comprising applying a crude mixture containing hG-CSF to an HPLC column and eluting hG-CSF from the column.

2. A process for the preparation of an hG-CSF mutein, the process comprising coupling together by the formation of peptide bonds successive amino acid residues, wherein at least one amino acid residue at a yeast KEX2 processing site in the native sequence has been altered to render the site inactive.

3. A process for the preparation of an hG-CSF mutein having an amino acid sequence which is substantially homologous to the synthetic amino acid sequence shown in Figure 1, the process comprising coupling together by the formation of peptide bonds successive amino acid residues.

4. A process for the preparation of an hG-CSF mutein, the process comprising coupling together by the formation of peptide bonds successive amino acid residues, wherein $Cys^{17}$ of the native sequence has been replaced by a non-Cys amino acid residue.

5. A process for the preparation of an hG-CSF mutein, the process comprising coupling together by the formation of peptide bonds successive amino acid residues, wherein $Phe^{83}$ of the native sequence has been replaced by a Cys residue.

6. A DNA segment encoding a human G-CSF protein, said segment characterized by increased deoxyadenosine and deoxythymidine content relative to the native hG-CSF sequence.

7. A DNA segment according to Claim 6, having substantial homology to the synthetic DNA sequence of FIG 1.

8. A DNA segment encoding encoding a protein according to any one of claims 2 to 5.

9. A crude culture supernatant comprising a protein according to any one of claims 1 to 3 at a protein concentration of at least 10 mcg/ml.

10. A recombinant yeast expression vector comprising a DNA segment encoding hG-CSF or an hG-CSF mutein.

11. A recombinant microbial expression vector comprising a DNA segment according to any one of claims 6 to 8.

12. A recombinant expression vector which is or is derived from pBC102.K22 (ATCC 67,255).

13. A process for preparing hG-CSF or an h-GSF mutein, comprising culturing a host microorganism comprising an expression vector according to any one of claims 10 to 12 under conditions promoting expression.

**FIG. 1: COMPARISON OF hG-CSF SEQUENCES**

N = Native hG-CSF; S = Synthetic sequence.

N CAG CTG CTG CTG TGG CAC AGT GCA CTC TGG ACA GTG CAG GAA GCG

↓

S ACT CCT CTG GGC CCT GCT TCT TCT CTG CCT CAA TCT TTT CTG CTC 45
N ACC CCC CTG GGC CCT GCC AGC TCC CTG CCC CAG AGC TTC CTG CTC 45
Thr Pro Leu Gly Pro Ala Ser Ser Leu Pro Gln Ser Phe Leu Leu 15

AGT ↓ Lys
S AAA TGT CTA GAA CAA GTT AAA AAA ATC CAG GGC GAT GGT GCA GCT 90
N AAG TGC TTA GAG CAA GTG AGG AAG ATC CAG GGC GAT GGC GCA GCG 90
Lys Cys Leu Glu Gln Val Arg Lys Ile Gln Gly Asp Gly Ala Ala 30
Ser

S CTT CAA GAA AAA CTG TGT GCT ACT TAT AAG CTT TGT CAT CCC GAG 135
N CTC CAG GAG AAG CTG TGT GCC ACC TAC AAG CTG TGC CAC CCC GAG 135
Leu Gln Glu Lys Leu Cys Ala Thr Tyr Lys Leu Cys His Pro Glu 45

GAG CTG GTG CTG CTC GGA CAC TCT CTG GGC ATC CCC TGG GCT CCC 180
Glu Leu Val Leu Leu Gly His Ser Leu Gly Ile Pro Trp Ala Pro 60

CTG AGC AGC TGC CCC AGC CAG GCC CTG CAG CTG GCA GGC TGC TTG 225
Leu Ser Ser Cys Pro Ser Gln Ala Leu Gln Leu Ala Gly Cys Leu 75

↓ TGC ↓
AGC CAA CTC CAT AGC GGC CTT TTC CTC TAC CAG GGG CTC CTG CAG 270
Ser Gln Leu His Ser Gly Leu Phe Leu Tyr Gln Gly Leu Leu Gln 90
Cys

GCT CTG GAA GGG ATA TCC CCT GAG CTC GGT CCT ACC TTG GAT ACA 315
Ala Leu Glu Gly Ile Ser Pro Glu Leu Gly Pro Thr Leu Asp Thr 105

CTG CAA CTG GAT GTT GCT GAT TTT GCT ACT ACT ATT TGG CAA CAA 360
Leu Gln Leu Asp Val Ala Asp Phe Ala Thr Thr Ile Trp Gln Gln 120

ATG GAA GAA CTG GGA ATG GCT CCT GCT CTG CAA CCT ACT CAA GGT 405
Met Glu Glu Leu Gly Met Ala Pro Ala Leu Gln Pro Thr Gln Gly 135

GCC ATG CCG GCT TTC GCG AGT GCT TTT CAA CGT CGT GCA GGA GGG 450
Ala Met Pro Ala Phe Ala Ser Ala Phe Gln Arg Arg Ala Gly Gly 150

GTC CTT GTT GCT TCT CAT CTG CAG AGT TTT CTT GAA GTT TCT TAT 495
Val Leu Val Ala Ser His Leu Gln Ser Phe Leu Glu Val Ser Tyr 165

CGT GTT CTA CGT CAT CTT GCT CAA CCA TGA AGG CCT CCA TGG GCA 540
Arg Val Leu Arg His Leu Ala Gln Pro End 174

2/2

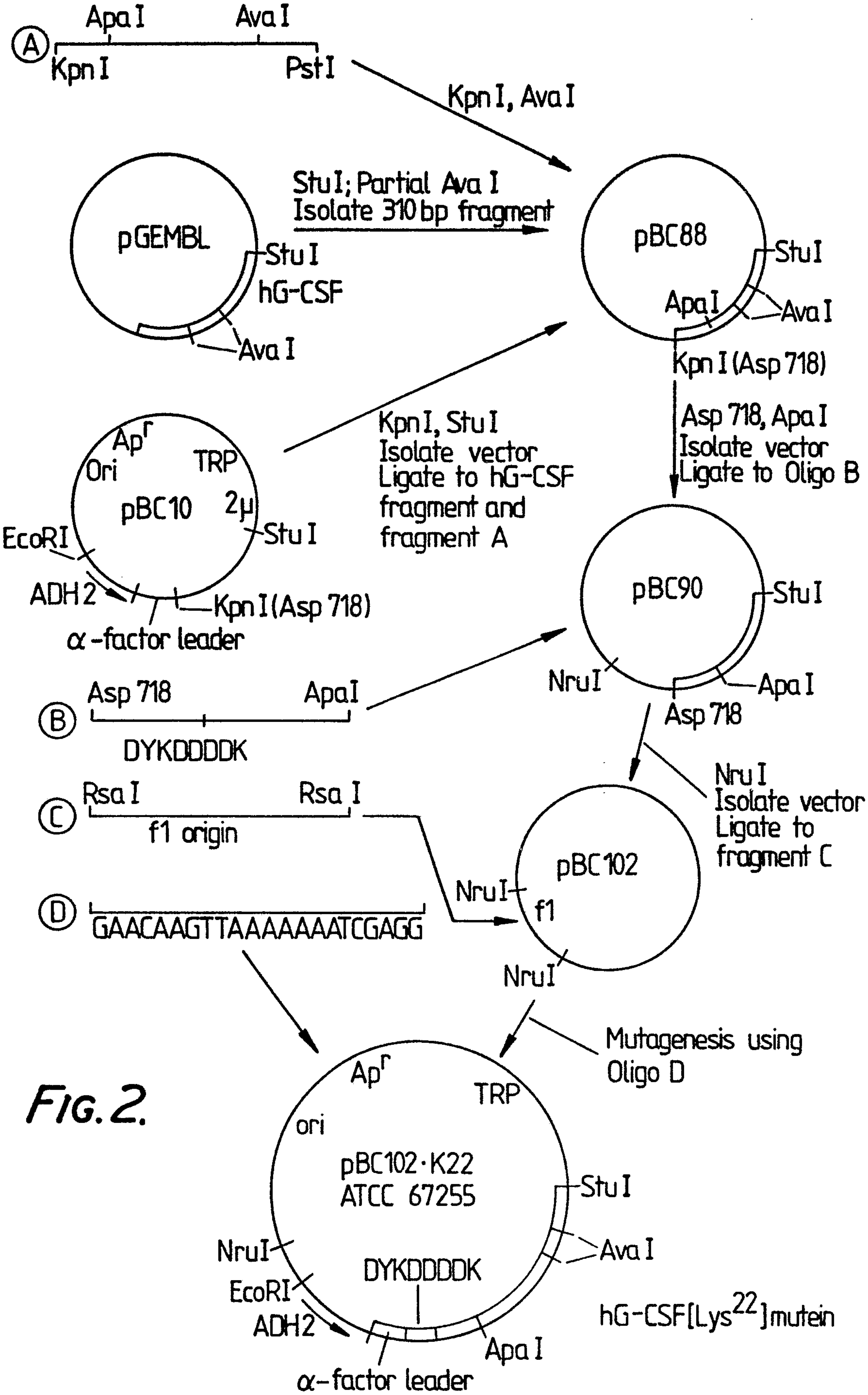
Ⓐ
Apa I
Ava I
Kpn I
Pst I
Kpn I, Ava I
pGEMBL
Stu I
hG-CSF
Ava I
Stu I; Partial Ava I
Isolate 310 bp fragment
pBC88
Stu I
Apa I
Ava I
Kpn I (Asp 718)
Asp 718, Apa I
Isolate vector
Ligate to Oligo B
Ap^r
Ori
TRP
pBC10
2μ
Stu I
EcoRI
ADH 2
Kpn I (Asp 718)
α-factor leader
Kpn I, Stu I
Isolate vector
Ligate to hG-CSF
fragment and
fragment A
pBC90
Stu I
Nru I
Apa I
Asp 718
Ⓑ
Asp 718
Apa I
DYKDDDDK
Nru I
Isolate vector
Ligate to
fragment C
Ⓒ
Rsa I
Rsa I
f1 origin
pBC102
Nru I
f1
Nru I
Ⓓ
GAACAAGTTAAAAAAATCGAGG
Mutagenesis using
Oligo D
Ap^r
TRP
ori
pBC102·K22
ATCC 67255
Stu I
Ava I
Nru I
DYKDDDDK
EcoRI
ADH2
Apa I
α-factor leader
hG-CSF[Lys^22]mutein

FIG. 2.